# FASCICULE DE BREVET EUROPEEN

(11) **EP 1 578 288 B1**
(45) Date de publication et mention de la délivrance du brevet: **15.08.2007**
(21) Numéro de dépôt: 03778390.9
(22) Date de dépôt: 14.10.2003
(51) Int. Cl.: A61B 17/70

(54) **DISPOSITIF D' IMMOBILISATION D'UNE TIGE DE LIAISON DANS UN ELEMENT D 'ANCRAGE OSSEUX D' UN IMPLANT RACHIDIEN**
VORRICHTUNG ZUM FESTHALTEN EINES VERBINDUNGSSTABES IN EINEM KNOCHENANKERELEMENT EINES WIRBELSÄULENIMPLANTATS
DEVICE FOR LOCKING A LINK ROD IN A BONE ANCHORING ELEMENT OF A SPINAL IMPLANT

(30) Priorité: 23.12.2002 FR 0216441; 17.07.2003 FR 0308701
(43) Date de publication de la demande: 28.09.2005
(73) Titulaire: Surgiview, 75002 Paris (FR)
(72) Inventeur: VIART, Guy, F-62128 Saint Leger (FR); ROKEGEM, Pascal, 62000 Arras (FR); LEROY, Jean-Yves, F-62870 Campagne Les Hesdin (FR); POMMIER, Arnaud, F-59283 Raimbeaucourt (FR); BERNARD, Pierre, 33700 Merignac (FR); LEPORT, Tiphaine, F-59800 Lille (FR)
(74) Mandataire: Schmitt, John
(86) Numéro de dépôt international: PCT/FR2003/003023
(87) Numéro de publication internationale: WO 2004/064653

(56) Documents cités:
- EP-A- 1 064 885
- EP-A- 1 190 678
- FR-A- 2 697 992
- FR-A- 2 729 291
- FR-A- 2 795 623
- US-A- 5 496 321
- US-A- 6 110 172

## Description

La présente invention est relative à un dispositif d'immobilisation d'une tige de liaison dans un élément d'ancrage osseux d'un implant rachidien.

On connaît différents types de dispositif d'immobilisation qui, du fait de leur structure particulière, permettent le blocage en rotation et en translation de la tige de liaison dans un élément d'ancrage osseux d'un implant rachidien.

Le document EP-A-1 064 885 décrit un dispositif d'immobilisation d'une tige de liaison d'un élément d'ancrage osseux selon le préambule de la revendication 1.

Le dispositif d'immobilisation suivant la présente invention a pour objet d'améliorer la retenue de l'élément de blocage sur l'élément d'ancrage osseux, tout en préservant des fixations indépendantes pour la retenue d'une part de la tige de liaison et d'autre de l'élément de blocage.

Le dispositif d'immobilisation suivant la présente invention comporte un élément d'ancrage osseux comprenant des moyens de retenue susceptibles de se déformer élastiquement sous un effort de poussée F et un élément de blocage comprenant d'une part des ergots qui coopèrent avec les moyens de retenue pour permettre la fixation de l'élément de blocage sur l'élément d'ancrage osseux et d'autre part, une vis de serrage permettant l'immobilisation en rotation et en translation de la tige de liaison entre l'élément d'ancrage osseux et l'élément de blocage.

Le dispositif d'immobilisation suivant la présente invention comprend :
❖ un élément d'ancrage osseux pourvu d'une tête comportant deux parois verticales délimitant une ouverture centrale en forme de U dont le fond présente un profil en portion de cylindre, chaque paroi verticale étant constituée d'une face centrale bordée latéralement et de chaque côté par des lames élastiques séparées respectivement de ladite face centrale par des fentes verticales, lesdites lames élastiques comportant respectivement dans leur partie supérieure une dent d'encliquetage,
❖ et un élément de blocage comportant un logement à profil en portion de cylindre, un alésage fileté débouchant à l'intérieur du logement, une vis de serrage coopérant avec l'alésage fileté et des ergots qui coopèrent respectivement avec une dent solidaire de lames élastiques.

Le dispositif d'immobilisation suivant la présente invention comporte un élément d'ancrage osseux dont la face centrale de chaque paroi verticale est percée d'un trou débouchant à l'intérieur de l'ouverture centrale en forme de U.

Le dispositif d'immobilisation suivant la présente invention comporte un élément d'ancrage osseux dont les lames élastiques de la tête comportent respectivement dans leur partie supérieure une dent dont le profil externe est bombé et incliné.

Le dispositif d'immobilisation suivant la présente invention comporte un élément de blocage dont la face inférieure comprend, suivant une direction parallèle à l'axe XX' de la tige de liaison, un logement présentant un profil en portion de cylindre.

Le dispositif d'immobilisation suivant la présente invention comporte un élément de blocage dont la face supérieure, opposée à celle inférieure, comprend en son milieu un alésage fileté débouchant à l'intérieur du logement et dans lequel coopère une vis de serrage.

Le dispositif d'immobilisation suivant la présente invention comporte un élément de blocage qui présente une première paire de faces latérales opposées comportant respectivement au dessus du logement une empreinte destinée à coopérer un instrument pour la manipulation et la mise en place dudit élément de blocage sur l'élément d'ancrage osseux.

Le dispositif d'immobilisation suivant la présente invention comporte un élément de blocage qui présente une seconde paire de faces latérales opposées qui sont solidaires chacune de deux ergots disposés dans la largeur dudit élément de blocage et positionnés dans le prolongement de la première paire de faces latérales.

Le dispositif d'immobilisation suivant la présente invention comporte un élément de blocage dont chaque ergot comprend respectivement dans sa partie supérieure un pan incliné ou chanfrein dont la base inférieure est positionnée dans le plan contenant chacune desdites premières paires de faces latérales.

Le dispositif d'immobilisation suivant la présente invention comporte un élément de blocage dont chaque ergot comprend respectivement dans sa partie inférieure et à l'opposé des pans inclinés, un profil arrondi.

Le dispositif d'immobilisation suivant la présente invention comporte un élément de blocage dont la distance **d** séparant deux ergots est inférieure à celle prévue entre deux dents d'une même paroi verticale de l'élément d'ancrage osseux.

Le dispositif d'immobilisation suivant la présente invention comporte des lames élastiques qui se déforment, sous une force de poussée **F** appliquée sur l'élément de blocage, latéralement en direction de la face centrale de chaque paroi de l'élément d'ancrage osseux.
Le dispositif d'immobilisation suivant la présente invention comprend :
- un élément d'ancrage osseux pourvu d'une tête comportant deux parois verticales tronquées délimitant une ouverture centrale en forme de U dont le fond présente un profil en portion de cylindre, chaque paroi verticale étant constituée d'une face centrale bordée latéralement et de chaque côté par des lames élastiques séparées respectivement de ladite face centrale par des fentes verticales, lesdites lames élastiques comportant respectivement dans leur partie supérieure une dent d'encliquetage,
- et un élément de blocage comportant un logement à profil en portion de cylindre, un alésage fileté débouchant à l'intérieur du logement, une vis de serrage coopérant avec l'alésage fileté et des ergots qui coopèrent respectivement avec une dent solidaire des lames élastiques.

Le dispositif d'immobilisation suivant la présente invention comporte une tête comprenant deux parois verticales à profil tronqué disposées l'une en face de l'autre et dans des plans parallèles afin de délimiter une première ouverture centrale en forme de U portée par l'axe XX' de la tige de liaison et dont le fond présente un profil en portion de cylindre et une seconde ouverture perpendiculaire à l'axe XX' et à la première ouverture.

Le dispositif d'immobilisation suivant la présente invention comporte une tête comprenant deux ouvertures perpendiculaires qui permettent de délimiter à chaque angle de la tête des lames élastiques susceptibles de se déformer élastiquement sous un effort de poussée F.

Le dispositif d'immobilisation suivant la présente invention comporte une tête pourvue de lames élastiques comprenant respectivement dans leur partie supérieure une dent dont le profil d'accrochage est tourné en direction de l'intérieur de la seconde ouverture et au-dessus de la face centrale de chaque paroi verticale.

Le dispositif d'immobilisation suivant la présente invention comporte une tête dont chaque dent comprend, au-dessus de sa partie d'accrochage et en direction de l'ouverture, un profil externe incliné se prolongeant en direction de l'extérieur par un profil bombé.

Le dispositif d'immobilisation suivant la présente invention comporte un élément de blocage comprenant une face inférieure comprenant suivant une direction parallèle à l'axe XX' un logement présentant un profil en portion de cylindre afin de coopérer avec la tige de liaison, une face supérieure comprenant en son milieu un alésage fileté débouchant à l'intérieur du logement et dans lequel coopère une vis de serrage, et des faces latérales parallèles deux à deux et dont deux au moins sont solidaires respectivement de deux ergots en forme de dent.

Le dispositif d'immobilisation suivant la présente invention comporte un élément de blocage dont chaque ergot comprend une partie d'accrochage positionnée en retrait et à une certaine distance d1 des faces latérales et opposées de l'élément de blocage.

Le dispositif d'immobilisation suivant la présente invention comprend :
- un élément d'ancrage osseux pourvu d'une tête comportant deux parois verticales délimitant une ouverture centrale en forme de U dont le fond présente un profil en portion de cylindre, chaque paroi verticale étant séparée du fond de l'ouverture centrale par une fente verticale donnant une certaine élasticité à chaque paroi selon une direction YY', lesdites parois verticales comprenant respectivement à chaque extrémité un profil en forme de lame d'accrochage élastique disposée l'une en face de l'autre et de part et d'autre de l'ouverture centrale, lesdites lames élastiques comportant respectivement dans leur partie supérieure une dent d'encliquetage,
- et un élément de blocage comportant un logement à profil en portion de cylindre, un alésage fileté débouchant à l'intérieur du logement, une vis de serrage coopérant avec l'alésage fileté et des ergots qui coopèrent respectivement avec une dent solidaire des lames élastiques.
   Le dispositif d'immobilisation suivant la présente invention comporte une tête dont chaque paroi verticale comprend sur sa face interne et entre les lames d'accrochage un logement vertical.

Le dispositif d'immobilisation suivant la présente invention comporte une tête dont les lames élastiques comprennent respectivement dans leur partie supérieure une dent dont le profil d'accrochage est tourné en direction de l'intérieur de l'ouverture centrale.

Le dispositif d'immobilisation suivant la présente invention comporte une tête dont chaque dent comprend au-dessus de sa partie d'accrochage et en direction de l'ouverture un profil externe incliné se prolongeant en direction de l'extérieur par un profil bombé.

Le dispositif d'immobilisation suivant la présente invention comporte un élément de blocage comprenant une face inférieure comprenant, suivant une direction parallèle à l'axe XX', un logement présentant un profil en portion de cylindre afin de coopérer avec la tige de liaison, une face supérieure comprenant en son milieu un alésage fileté débouchant à l'intérieur du logement et dans lequel coopère une vis de serrage et des faces latérales parallèles deux à deux et dont deux au moins sont solidaires respectivement de deux ergots en forme de dent.

Le dispositif d'immobilisation suivant la présente invention comporte un élément de blocage dont chaque face latérale disposée dans un plan parallèle à l'axe XX' du logement comporte deux ergots en forme de dent séparés par une nervure verticale présentant un logement central vertical.

Le dispositif d'immobilisation suivant la présente invention comporte un élément de blocage dont les parties d'accrochage des ergots sont fermées à l'opposé des faces latérales par l'intermédiaire de la nervure verticale correspondante.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en perspective éclatée illustrant le dispositif d'immobilisation suivant la présente invention.
Figure 2 est une vue en perspective montrant l'élément d'ancrage osseux du dispositif d'immobilisation suivant la présente invention.
Figure 3 est une vue en perspective représentant l'élément de blocage en translation et en rotation de la tige de liaison à l'intérieur de l'élément d'ancrage osseux du dispositif d'immobilisation suivant la présente invention.
Figure 4 est une vue en perspective illustrant la déformation élastique de l'élément d'ancrage osseux lors du montage de l'élément de blocage du dispositif d'immobilisation suivant la présente invention.
Figure 5 est une vue en perspective montrant le dispositif d'immobilisation en position assemblée pour le blocage en rotation et en translation de la tige de liaison de l'implant rachidien.
Figure 6 est une vue en perspective éclatée illustrant une première variante du dispositif d'immobilisation suivant la présente invention.
Figure 7 est une vue en perspective montrant l'élément d'ancrage osseux de la première variante du dispositif d'immobilisation suivant la présente invention.
Figure 8 est une vue en perspective représentant l'élément de blocage en translation et en rotation de la tige de liaison à l'intérieur de l'élément d'ancrage osseux de la première variante du dispositif d'immobilisation suivant la présente invention.
Figure 9 est une vue en perspective illustrant la déformation élastique de l'élément d'ancrage osseux lors du montage de l'élément de blocage de la première variante du dispositif d'immobilisation suivant la présente invention.
Figure 10 est une vue en perspective montrant la première variante du dispositif d'immobilisation en position assemblée pour le blocage en rotation et en translation de la tige de liaison de l'implant rachidien.
Figure 11 est une vue en perspective éclatée illustrant une seconde variante du dispositif d'immobilisation suivant la présente invention.
Figure 12 et 13 sont des vues en perspective montrant l'élément d'ancrage osseux de la seconde variante du dispositif d'immobilisation suivant la présente invention.
Figure 14 est une vue en perspective représentant l'élément de blocage en translation et en rotation de la tige de liaison à l'intérieur de l'élément d'ancrage osseux de la seconde variante du dispositif d'immobilisation suivant la présente invention.
Figures 15 et 16 sont des vues illustrant la déformation élastique de l'élément d'ancrage osseux lors du montage de l'élément de blocage de la seconde variante du dispositif d'immobilisation suivant la présente invention.
Figure 17 et 18 sont des vues en perspective montrant une seconde variante du dispositif d'immobilisation en position assemblée pour le blocage en rotation et en translation de la tige de liaison de l'implant rachidien.

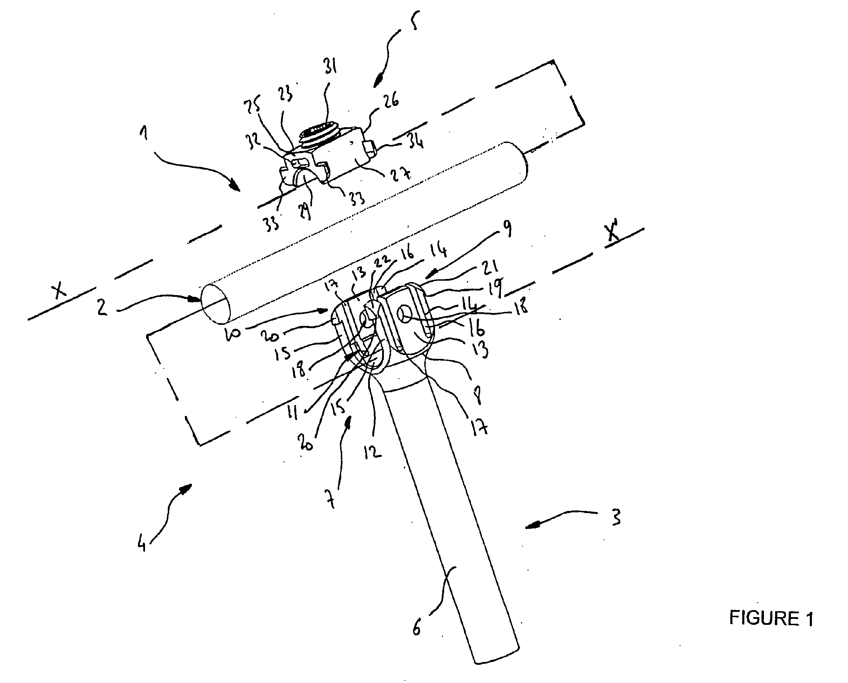

On a montré en figure 1 un dispositif d'immobilisation 1 d'un implant rachidien 4 pour le blocage en rotation et en translation d'une tige de liaison 2 au niveau de chaque vertèbre instrumentée d'une colonne vertébrale.

Le dispositif d'immobilisation 1 est constitué d'un élément d'ancrage osseux 3 et d'un élément de blocage 5 destiné à coopérer avec l'élément d'ancrage 3 pour la fixation en rotation et en translation de la tige de liaison 2.

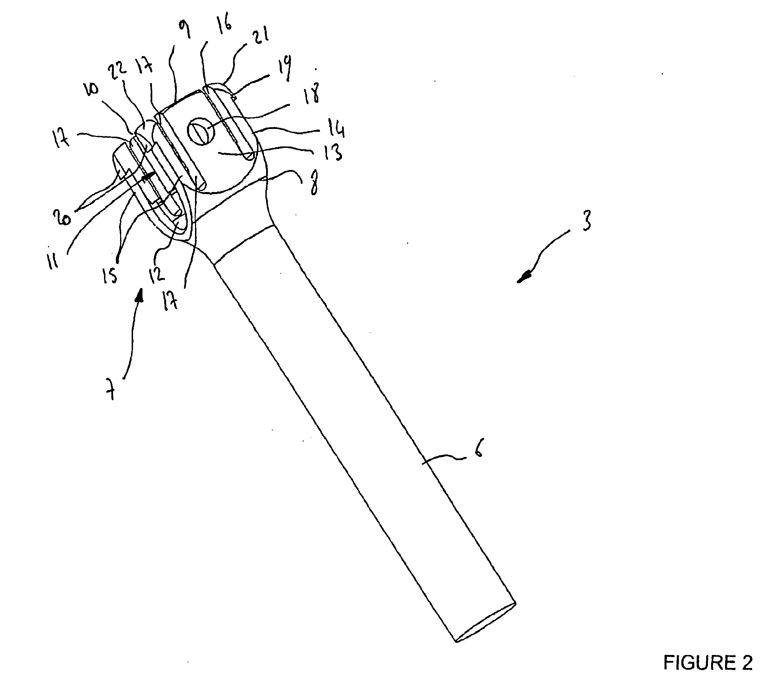

On a représenté en figure 2 l'élément d'ancrage osseux 3 comprenant une partie d'ancrage 6 et une partie de réception 7. La partie d'ancrage 6 peut présenter soit la forme d'un crochet, soit un profil fileté solidaire ou non de la partie de réception 7 pour venir se fixer sur/ou dans le corps vertébral de la vertèbre à instrumenter.

La partie de réception 7 est constituée d'une tête 8 en forme de U ouverte dans sa partie supérieure 7 pour pouvoir coopérer avec la tige de liaison 2 et l'élément de blocage 5.

La tête 8 comporte deux parois verticales 9, 10 disposées l'une en face de l'autre et dans des plans parallèles afin de délimiter une ouverture centrale 11 en forme de U dont le fond 12 présente un profil en portion de cylindre.

Chaque paroi verticale 9, 10 est constituée d'une face centrale 13 bordée latéralement et de chaque côté par des lames élastiques 14, 15 séparées respectivement de ladite face centrale par des fentes verticales 16, 17.

La face centrale 13 de chaque paroi verticale 9, 10 est percée d'un trou 18 débouchant à l'intérieur de l'ouverture centrale 11 en forme de U.

Les lames élastiques 14, 15 de la tête 8 comportent respectivement dans leur partie supérieure une dent 19, 20 dont le profil externe 21, 22 est bombé et incliné en direction de l'extérieur de chaque paroi verticale 9, 10.

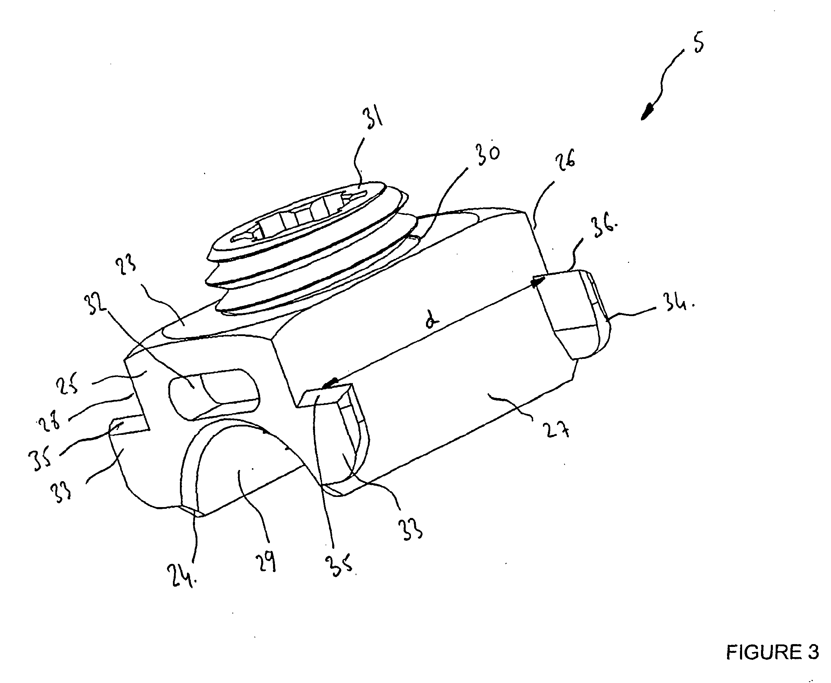

On a montré en figure 3 l'élément de blocage 5 du dispositif d'immobilisation 1 qui présente un profil externe sensiblement parallélépipédique dont chacune des faces opposées 23, 24; 25, 26, 27 et 28 sont parallèles deux à deux.

Ainsi la face inférieure 24 de l'élément de blocage 5 comporte suivant une direction parallèle à l'axe XX' de la tige de liaison 2 un logement 29 présentant un profil en portion de cylindre.

La face supérieure 23 de l'élément de blocage 5 comporte en son milieu un alésage fileté 30 débouchant à l'intérieur du logement 29 et dans lequel coopère une vis de serrage 31.

La première paire de faces latérales 25, 26 de l'élément de blocage 5 comporte respectivement au dessus du logement 29 une empreinte 32 destinée à coopérer avec les dents d'un instrument, non représentées, permettant la manipulation et la mise en place dudit élément de blocage sur l'élément d'ancrage osseux 3.

Les secondes paires de faces latérales 27, 28 de l'élément de blocage 5 sont solidaires chacune de deux ergots 33, 34 disposés dans la largeur dudit élément de blocage soit dans le prolongement de chaque face latérale 25, 26.

Ainsi, l'élément de blocage 5 comporte quatre ergots 33, 34 s'étendent en direction de l'extérieur de ce dernier et suivant une direction perpendiculaire au plan contenant chaque face latérale 27, 28.

Chaque ergot 33, 34 comporte respectivement dans sa partie supérieure un pan incliné ou chanfrein 35, 36 dirigé en direction des faces latérales 25, 26 de manière que la base inférieure de chaque pan incliné 35, 36 soit dans le plan contenant chacune desdites faces latérales 25, 26.

Chaque ergot 33, 34 comporte respectivement dans sa partie inférieure et à l'opposé des pans inclinés 35, 36 un profil arrondi 37, 38 permettant le glissement desdits ergots sur les dents 19, 20 lors de l'assemblage de l'élément de blocage 5 avec l'élément d'ancrage osseux 3.

La distance **d** prévue entre deux ergots 33, 34 d'une même face latérale 27, 28 est inférieure à celle prévue entre deux dents 19, 20 d'une même paroi verticale 9, 10 de la tête 8 de l'élément d'ancrage osseux 3.

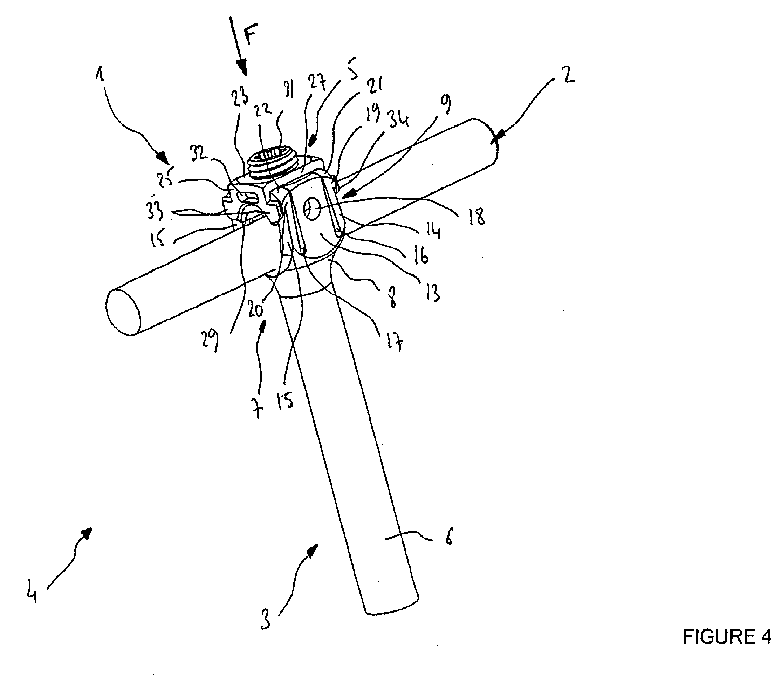

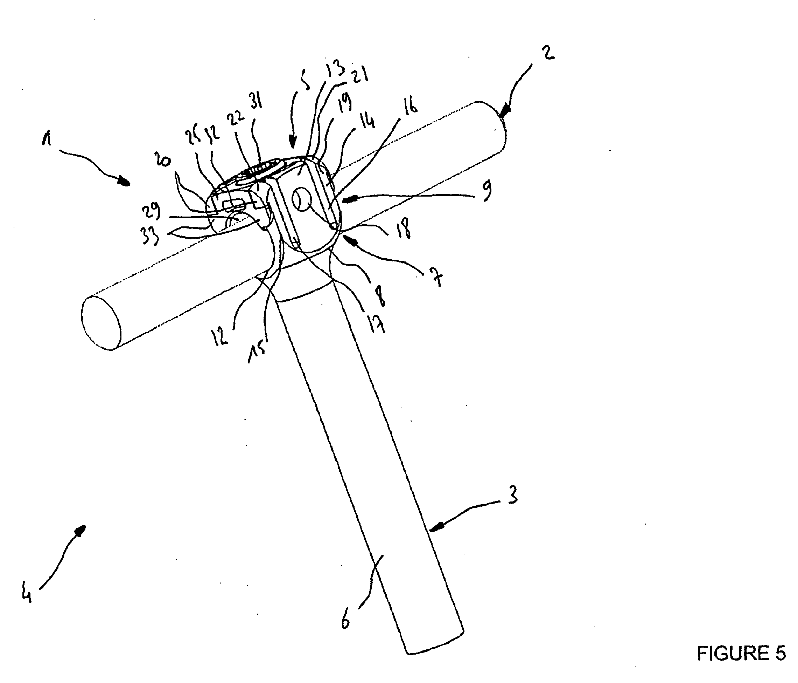

On a illustré en figures 4 et 5 la mise en place et la retenue de l'élément de blocage 5 sur la tête 8 de l'élément d'ancrage 3 afin de pouvoir bloquer en rotation et en translation la tige de liaison 2 dans chaque dispositif d'immobilisation 1 ancré de le corps vertébral d'une vertèbre.

L'élément d'ancrage osseux 3 est fixé ou accroché en fonction de sa structure au corps vertébral d'une vertèbre à instrumenter.

La tige de liaison 2 est positionnée à l'intérieur de l'ouverture centrale 11 de la tête 8 de l'élément d'ancrage osseux 3.

L'élément de blocage 5 est positionné au dessus de la tête 8 de l'élément d'ancrage osseux 3 de manière que les ergots 33, 34 d'une même face latérale 27, 28 viennent en appui contre les dents correspondantes 19, 20 d'une même paroi verticale 9, 10.

Une force de poussée F est appliquée à l'aide d'un instrument, non représenté, sur l'élément de blocage 5 afin que les ergots 33, 34 de chaque face latérale 27, 28 déforme latéralement les lames élastiques 14, 15 de chaque paroi 9, 10 de la tête 8 de l'élément d'ancrage osseux 3.

La déformation élastique des lames 14, 15 s'effectue en direction de la face centrale 13 de chaque paroi verticale 9, 10 de la tête 8 du fait de la différence de largeur prévue entre les ergots 33, 34 et les dents 19, 20 (figure 4).

L'introduction de l'élément de blocage 5 est facilitée par le fait que chaque ergot 33, 34 présente une partie inférieure à profil arrondi 37, 38 qui glisse sur le profil externe bombé 21, 22 de chaque dent 19, 20 solidaire des lames 14, 15.

La force de poussée F doit être suffisante pour que chaque ergot 33, 34 vienne s'encliqueter avec la dent 19, 20 correspondante des lames élastiques 14, 15. La retenue des ergots 33, 34 est obtenue lorsque chaque pan incliné 35, 36 coopère avec le profil de la dent 19, 20 correspondante (figure 5).

Egalement, la retenue des ergots 33, 34 est obtenue par l'élasticité des lames 14, 15 qui reviennent en position de repos après le passage des ergots 33, 34 sur les dents 19, 20 correspondantes.

La tige de liaison 2 est ensuite immobilisée en rotation et en translation par l'intermédiaire de la vis de serrage 31 qui est vissée à l'intérieur de l'alésage 30 de l'élément de blocage 5. La vis de serrage 31, sous l'effort de vissage, vient bloquer la tige de liaison 2 contre le fond 12 en portion de cylindre de l'ouverture centrale 11 de la tête 8 de l'élément d'ancrage 3.

Egalement, l'effort de serrage de la vis de pression 31 contre la tige de liaison 2 permet, par l'intermédiaire d'un déplacement vertical dirigé suivant une direction opposée à celle de ladite tige, de bloquer l'élément de blocage 5 dans la tête 8 de l'élément d'ancrage 3.

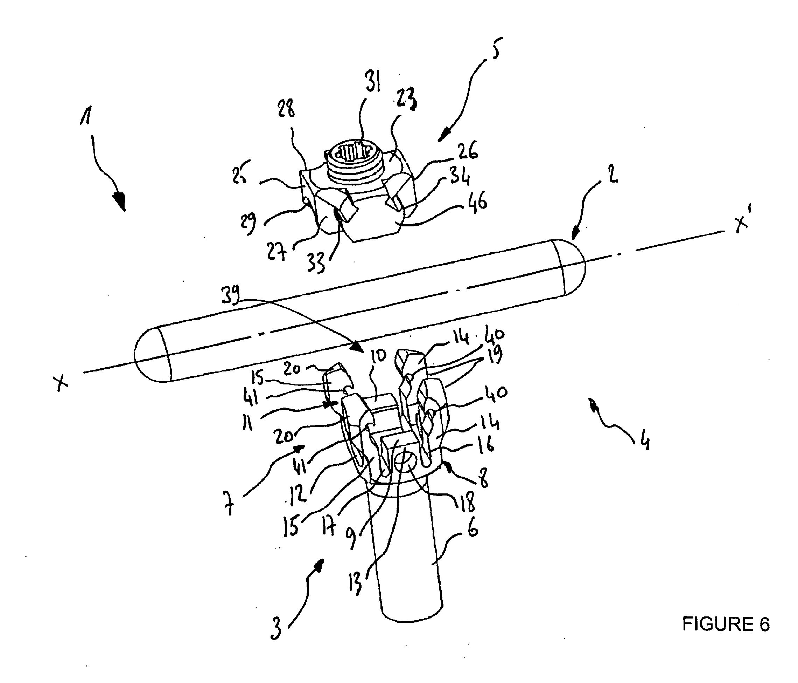

On a montré en figures 6 à 10 une première variante de l'implant rachidien et plus particulièrement du dispositif d'immobilisation 1 pour le blocage en rotation et en translation de la tige de liaison 2 au niveau de chaque vertèbre instrumentée d'une colonne vertébrale.

Par souci de clarté, les éléments identiques à ceux décrits précédemment présentent la même référence afin d'éviter toute confusion.

Le dispositif d'immobilisation 1 est constitué d'un élément d'ancrage osseux 3 et d'un élément de blocage 5 destiné à coopérer avec l'élément d'ancrage 3 pour la fixation en rotation et en translation de la tige de liaison 2.

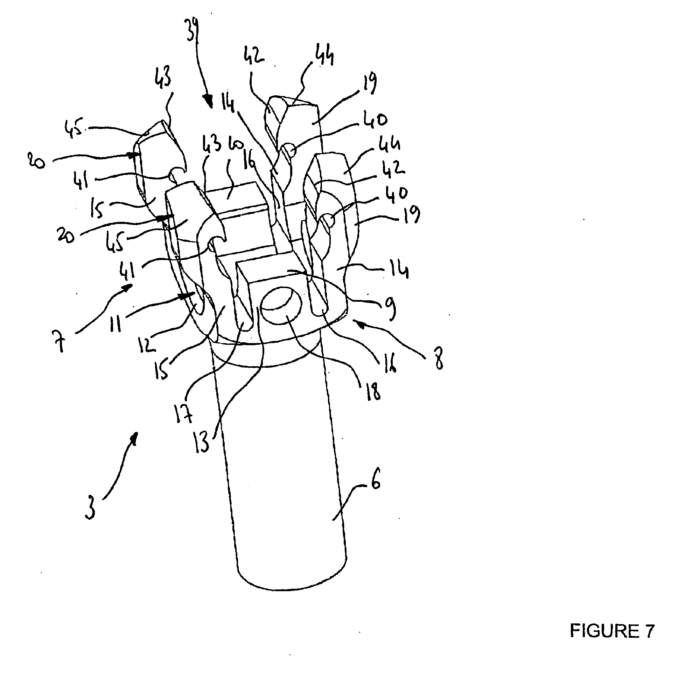

On a représenté en figure 7 l'élément d'ancrage osseux 3 comprenant une partie d'ancrage 6 et une partie de réception 7. La partie d'ancrage 6 peut présenter soit la forme d'un crochet, soit un profil fileté solidaire ou non de la partie de réception 7 pour venir se fixer sur/ou dans le corps vertébral de la vertèbre à instrumenter.

La partie de réception 7 est constituée d'une tête 8 en forme de U, ouverte dans sa partie supérieure pour pouvoir coopérer avec la tige de liaison 2 et l'élément de blocage 5.

La tête 8 comporte deux parois verticales 9, 10 à profil tronqué, disposées l'une en face de l'autre et dans des plans parallèles afin de délimiter une première ouverture centrale 11 en forme de U portée par l'axe XX' de la tige de liaison 2 et dont le fond 12 présente un profil en portion de cylindre et une seconde ouverture 39 perpendiculaire à l'axe XX' et à la première ouverture 11.

Les deux ouvertures perpendiculaires 11 et 39 permettent de délimiter à chaque angle de la tête 8 des moyens de retenue 14, 15 susceptibles de se déformer élastiquement sous un effort de poussée F.

Chaque paroi verticale tronquée 9, 10 est constituée d'une face centrale 13 dont la hauteur est délimitée par la seconde ouverture 39 traversant la tête 8 de la partie de réception 7.

Chaque face centrale 13 est bordée latéralement et de chaque côté par des lames élastiques 14, 15 séparées respectivement de ladite face centrale par des fentes verticales 16, 17.

La face centrale 13 de chaque paroi verticale tronquée 9, 10 est percée d'un trou 18 non débouchant permettant à une instrumentation de venir s'accrocher pour permettre l'introduction de l'élément de blocage 5 dans l'élément d'ancrage 3.

Les lames élastiques 14, 15 de la tête 8 comportent respectivement dans leur partie supérieure une dent 19, 20 dont le profil d'accrochage 40, 41 est tourné en direction de l'intérieur de la seconde ouverture 39 et au-dessus de la face centrale 13 de chaque paroi verticale 9, 10.

Chaque dent 19, 20 comporte au-dessus de sa partie d'accrochage 40, 41 et en direction de l'ouverture 39 un profil externe incliné 42, 43 se prolongeant en direction de l'extérieur de chaque lame 14, 15 par un profil bombé 44, 45.

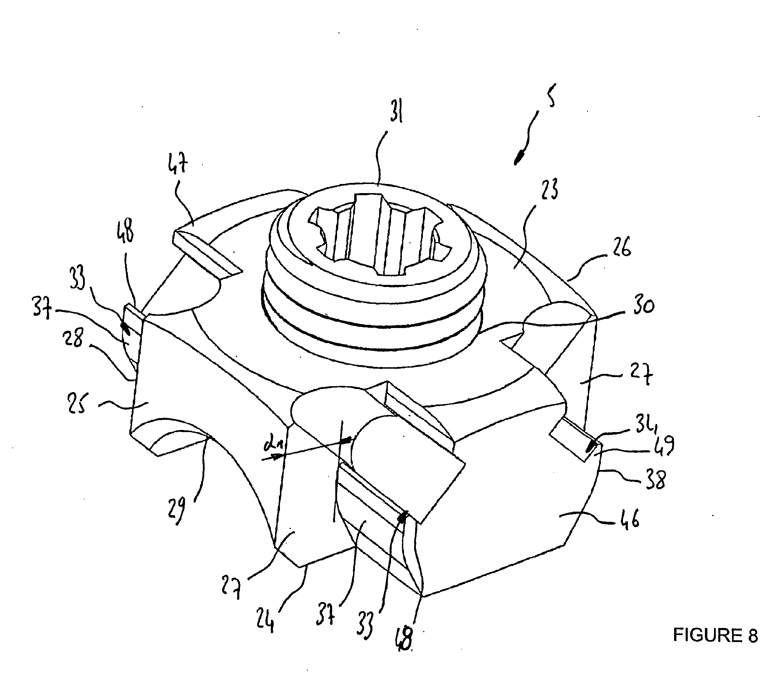

On a montré en figure 8 l'élément de blocage 5 du dispositif d'immobilisation 1 qui présente un profil externe sensiblement parallélépipédique dont chacune des faces opposées 23, 24; 25, 26, 27 et 28 sont parallèles deux à deux.

Ainsi, la face inférieure 24 de l'élément de blocage 5 comporte suivant une direction parallèle à l'axe XX' un logement 29 présentant un profil en portion de cylindre afin de recevoir la tige de liaison 2 lors du montage et de la fixation du dispositif d'immobilisation 1.

La face supérieure 23 de l'élément de blocage 5 comporte en son milieu un alésage fileté 30 débouchant à l'intérieur du logement 29 et dans lequel coopère une vis de serrage 31.

Chaque face latérale 27, 28 disposée dans un plan parallèle à l'axe XX' du logement 29 et perpendiculaire à chacune des faces latérales 25, 26 de l'élément de blocage 5, est solidaire d'une surépaisseur 46, 47 délimitant deux ergots 33, 34 en forme de dent.

Ainsi, l'élément de blocage 5 comporte deux ergots 33 et deux ergots 34 qui s'étendent en direction de l'extérieur de ce dernier.

Chaque ergot 33, 34 comporte respectivement une partie d'accrochage 48, 49 délimitée par un agencement de profils inclinés et bombés permettant une coopération avec les parties d'accrochage 40, 41 de chaque dent 19, 20 lors de la mise en place de l'élément de blocage 5 dans la tête 8 de l'élément d'ancrage 3.

On note que les parties d'accrochage 48, 49 de chaque ergot 33, 34 sont positionnées en retrait et à une distance **d1** des faces latérales et opposées 25, 26 de l'élément de blocage 5.

Chaque ergot 33, 34 présente respectivement un profil externe incliné 37, 38 permettant le glissement desdits ergots et l'écartement des lames élastiques 14, 15 vers l'extérieur de la tête 8 afin de pouvoir réaliser l'assemblage de l'élément de blocage 5 avec l'élément d'ancrage osseux 3.

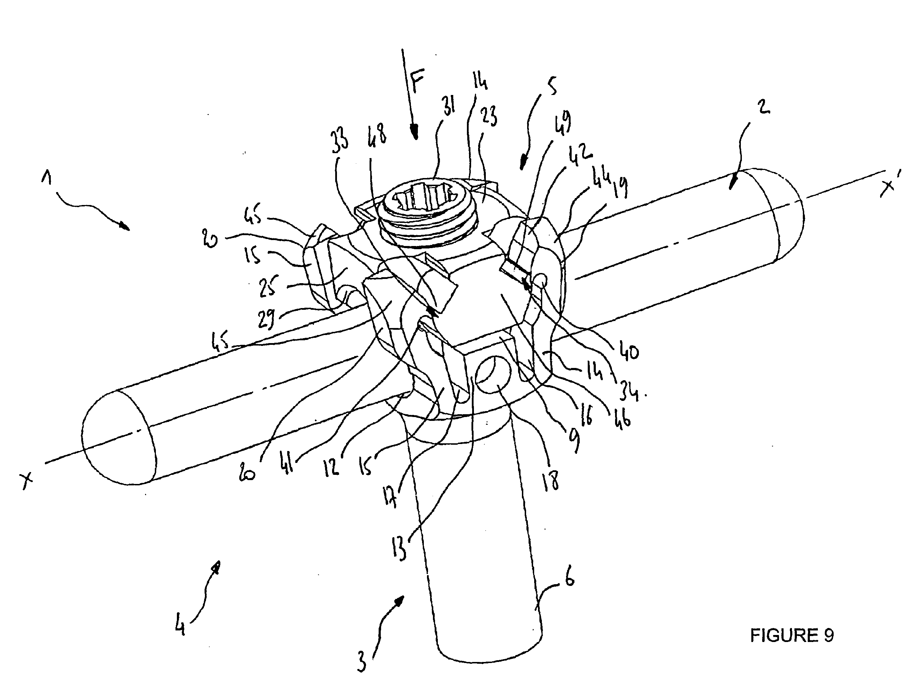

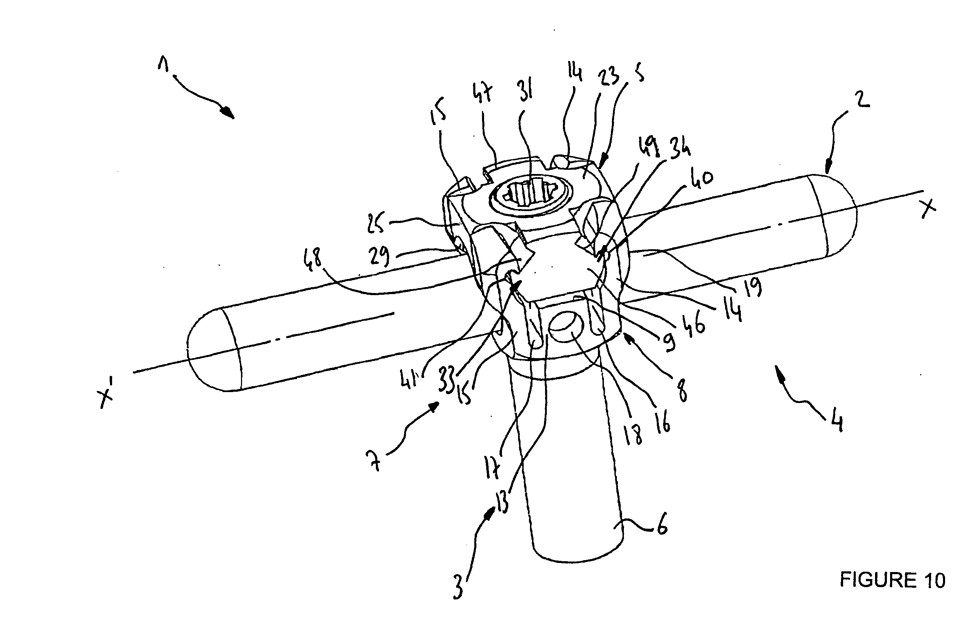

On a montré en figures 9 et 10 les différentes étapes permettant l'assemblage de l'élément de blocage 5 dans la tête 8 de l'élément d'ancrage 3 en vue de la fixation en rotation et en translation de la tige de liaison 2 dans chaque dispositif d'immobilisation 1 ancré dans le corps vertébral d'une vertèbre.

L'élément d'ancrage osseux 3 est fixé ou accroché en fonction de sa structure à une vertèbre à instrumenter.

La tige de liaison 2 est positionnée à l'intérieur de l'ouverture centrale 11 de la tête 8 de l'élément d'ancrage osseux 3 avant l'introduction de l'élément de blocage 5.

L'élément de blocage 5 est positionné au-dessus de la tête 8 de l'élément d'ancrage osseux 3 de manière que les ergots 33, 34 d'une même face latérale 27, 28 viennent en appui contre les dents correspondantes 19, 20 d'une même paroi verticale 9, 10.

Une force de poussée **F** est effectuée suivant une direction sensiblement verticale à l'aide d'un instrument, non représenté, sur l'élément de blocage 5 afin que les ergots 33, 34 de chaque face latérale 27, 28 déforment latéralement les lames élastiques 14, 15 de chaque paroi 9, 10 de la tête 8 de l'élément d'ancrage osseux 3.

La déformation élastique des lames 14, 15 s'effectue en direction de l'extérieur de la tête 8, c'est à dire suivant une direction qui s'éloigne de la face centrale 13 de chaque paroi verticale 9, 10 de la tête 8, du fait de la différence des dimensions prévues entre les ergots 33, 34 et les dents 19, 20 (figure 9).

L'introduction de l'élément de blocage 5 est facilitée par le fait que chaque ergot 33, 34 présente une partie inférieure à profil incliné 37, 38 qui glisse sur le profil externe de chaque dent 19, 20 solidaire des lames 14, 15.

La force de poussée **F** doit être suffisante pour que chaque partie d'accrochage 48, 49 des ergots 33, 34 vienne s'encliqueter avec la partie d'accrochage 40, 41 de chaque dent 19, 20 correspondante des lames élastiques 14, 15.

La retenue de l'élément de blocage 5 est obtenue par l'élasticité des lames 14, 15 qui reviennent en position de repos après le passage des ergots 33, 34 sur les dents 19, 20 correspondantes.

La tige de liaison 2 est ajustée par coulissement dans une gouttière cylindrique constituée par le fond 12 présentant un profil en portion de cylindre de la tête 8 de l'élément d'ancrage osseux 3 et le logement 29 présentant un profil en portion de cylindre de l'élément de blocage 5.

La tige de liaison 2 est ensuite immobilisée en rotation et en translation par l'intermédiaire de la vis de serrage 31 qui est vissée à l'intérieur de l'alésage 30 de l'élément de blocage 5. La vis de serrage 31, sous l'effort de vissage, vient bloquer la tige de liaison 2 contre le fond 12 en portion de cylindre de l'ouverture centrale 11 de la tête 8 de l'élément d'ancrage 3.

Egalement, l'effort de serrage de la vis de pression 31 contre la tige de liaison 2 permet, par l'intermédiaire d'un déplacement vertical dirigé suivant une direction opposée à celle de ladite tige, de bloquer l'élément de blocage 5 dans la tête 8 de l'élément d'ancrage 3.

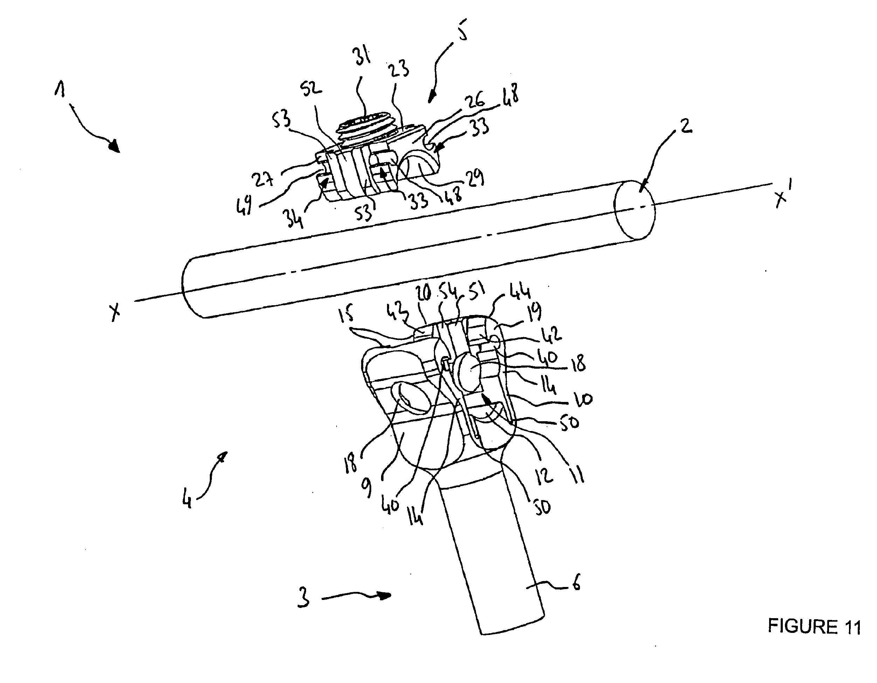

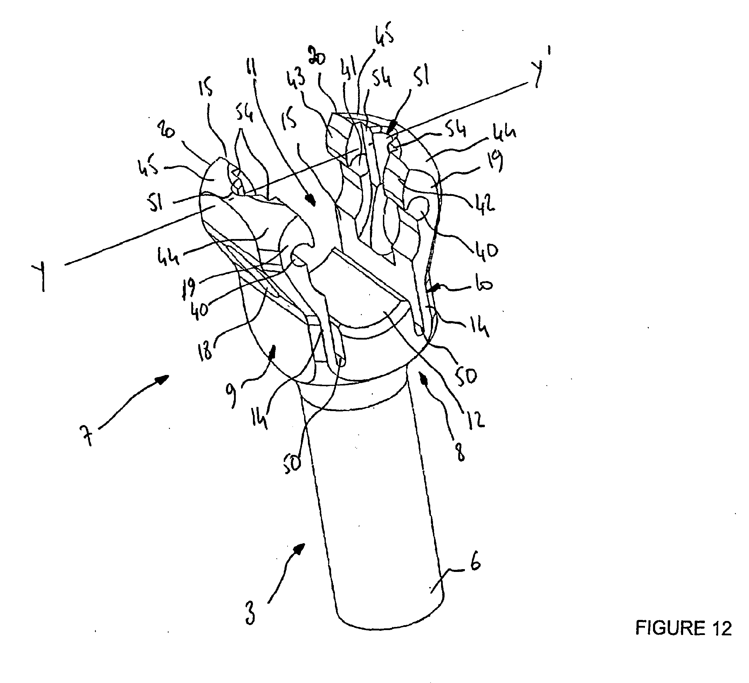

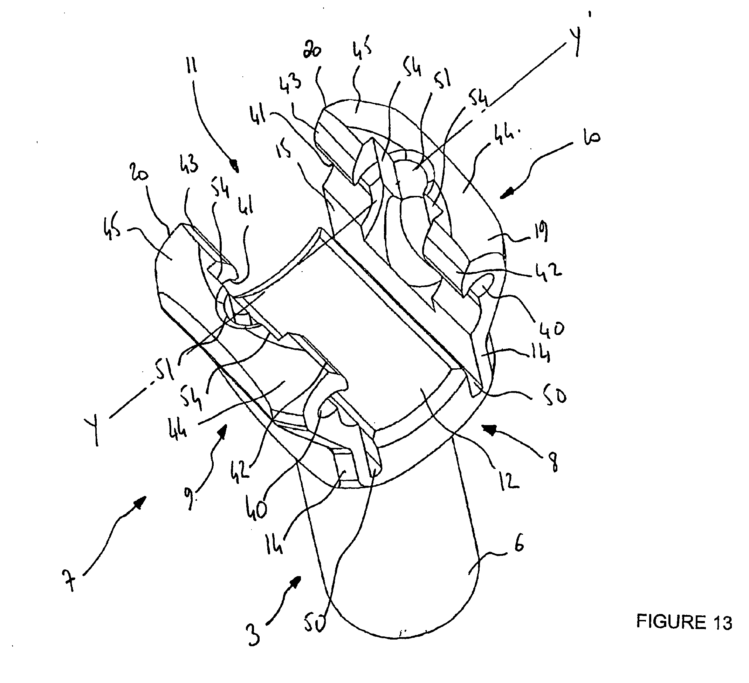

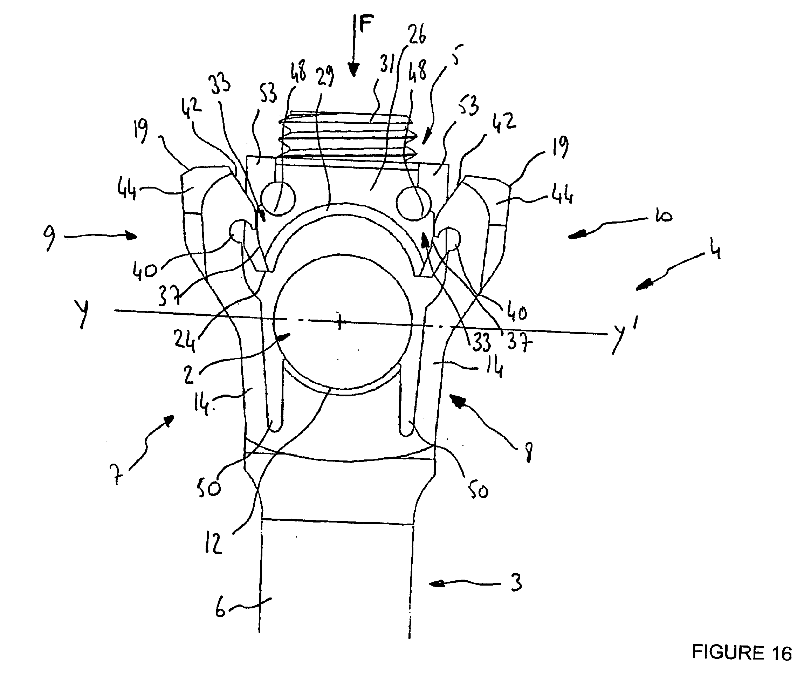

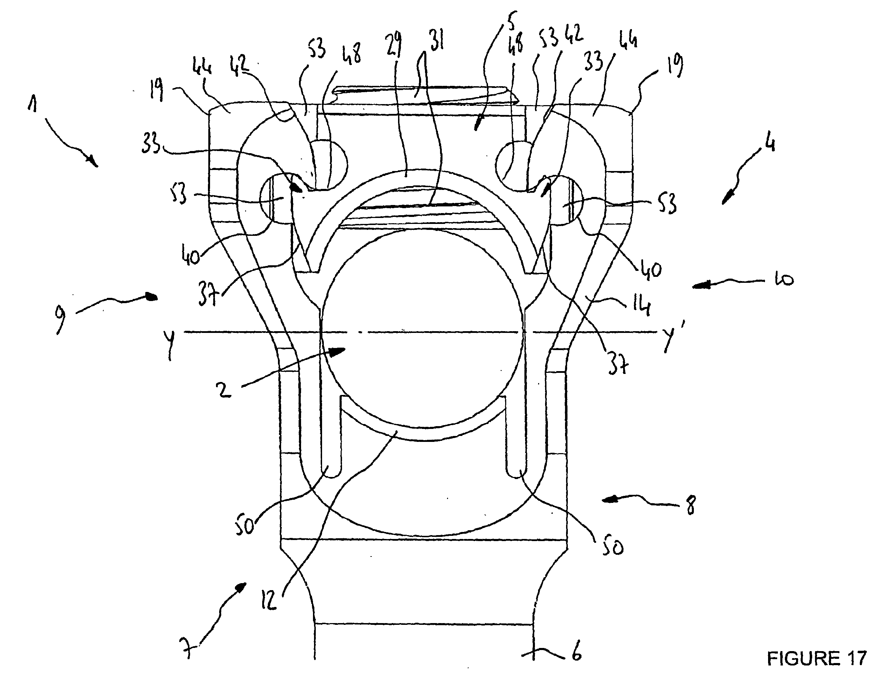

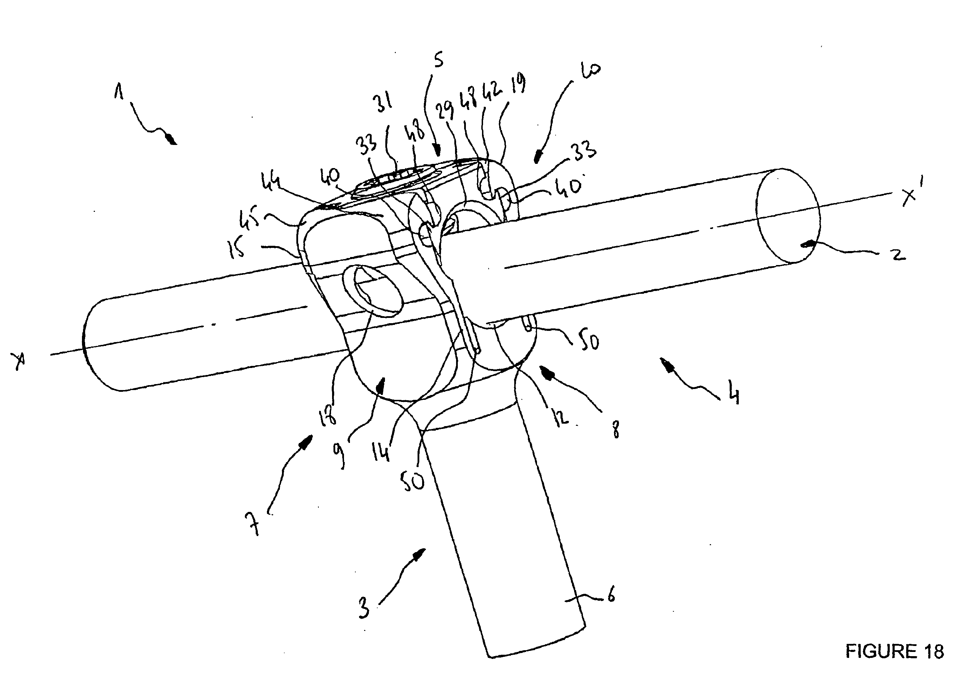

On a montré en figure 11 à 18 une seconde variante de l'implant rachidien et plus particulièrement du dispositif d'immobilisation 1 pour le blocage en rotation et en translation d'une tige de liaison 2 au niveau de chaque vertèbre instrumentée d'une colonne vertébrale.

Par souci de clarté, les éléments identiques à ceux décrits précédemment présentent les mêmes références.

Le dispositif d'immobilisation 1 est constitué d'un élément d'ancrage osseux 3 et d'un élément de blocage 5 destinés à coopérer avec l'élément d'ancrage 3 pour la fixation en rotation et en translation de la tige de liaison 2.

L'élément d'ancrage osseux 3 comprenant une partie d'ancrage 6 et une partie de réception 7. La partie d'ancrage 6 peut présenter soit la forme d'un crochet, soit un profil fileté solidaire ou non de la partie de réception 7 pour venir se fixer sur/ou dans la vertèbre à instrumenter.

La partie de réception 7 est constituée d'une tête 8 en forme de U ouverte dans sa partie supérieure pour pouvoir coopérer avec la tige de liaison 2 et l'élément de blocage 5.

La tête 8 comporte deux parois verticales 9, 10 disposées l'une en face de l'autre et dans des plans parallèles afin de délimiter une première ouverture centrale 11 en forme de U portée par l'axe XX' de la tige de liaison 2 et dont le fond 12 présente un profil en portion de cylindre.

Chaque paroi verticale 9, 10 est séparée du fond 12 de l'ouverture centrale 11 par une fente verticale 50 donnant une certaine élasticité à chaque paroi suivant une direction YY' perpendiculaire à celle XX' de la tige de liaison 2.

Les parois verticales élastiques 9, 10 comportent respectivement à chaque extrémité un profil en forme de lame d'accrochage 14, 15 disposée l'une en face de l'autre et de part et d'autre de l'ouverture centrale 11.

Chaque paroi verticale élastique 9, 10 comporte sur sa face interne et entre les lames d'accrochage 14, 15 un logement vertical 51 présentant un profil en portion de cylindre.

Le logement vertical 51 possède de chaque côté une rainure 54 permettant de guider l'élément de blocage 5 lors de sa mise en place à l'intérieur de la tête 8 en forme de U.

Chaque paroi verticale élastiques 9, 10 est percée entre les lames d'accrochage 14, 15 d'un trou 18 débouchant à l'intérieur de l'ouverture centrale 11 permettant à une instrumentation de venir s'accrocher pour permettre l'introduction de l'élément de blocage 5 dans l'élément d'ancrage 3.

Les lames d'accrochage 14, 15 de la tête 8 comportent respectivement dans leur partie supérieure une dent 19, 20 dont le profil d'accrochage 40, 41 est tourné en direction de l'intérieur de l'ouverture centrale 11.

Chaque dent 19, 20 comporte au-dessus de sa partie d'accrochage 40, 41 un profil externe incliné 42, 43 se prolongeant en direction de l'extérieur de chaque lame 14, 15 par un profil bombé 44, 45.

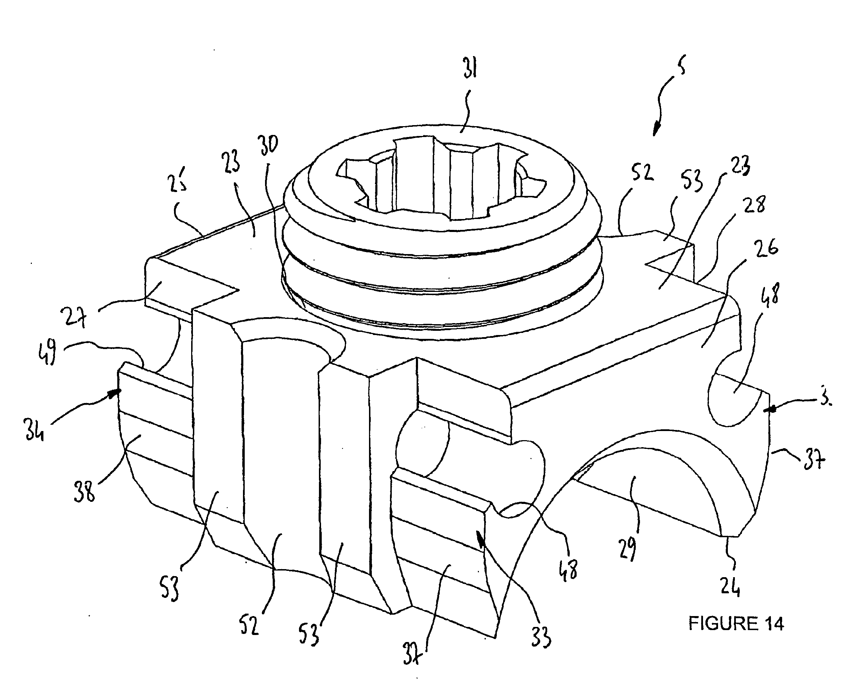

On a montré en figure 14 l'élément de blocage 5 du dispositif d'immobilisation 1 qui présente un profil externe sensiblement parallélépipédique dont chacune des faces opposées 23, 24; 25, 26, 27 et 28 sont parallèles deux à deux.

Ainsi la face inférieure 24 de l'élément de blocage 5 comporte suivant une direction parallèle à l'axe XX' un logement 29 présentant un profil en portion de cylindre afin de recevoir la tige de liaison 2 lors du montage et de la fixation du dispositif d'immobilisation 1.

La face supérieure 23 de l'élément de blocage 5 comporte en son milieu un alésage fileté 30 débouchant à l'intérieur du logement 29 et dans lequel coopère une vis de serrage 31.

Chaque face latérale 27, 28 disposée dans un plan parallèle à l'axe XX' du logement 29 et perpendiculaire à chacune des faces latérales 25, 26 de l'élément de blocage 5, comporte deux ergots 33, 34 en forme de dent.

Ainsi, l'élément de blocage 5 comporte deux ergots 33 et deux ergots 34 qui s'étendent en direction de l'extérieur de ce dernier.

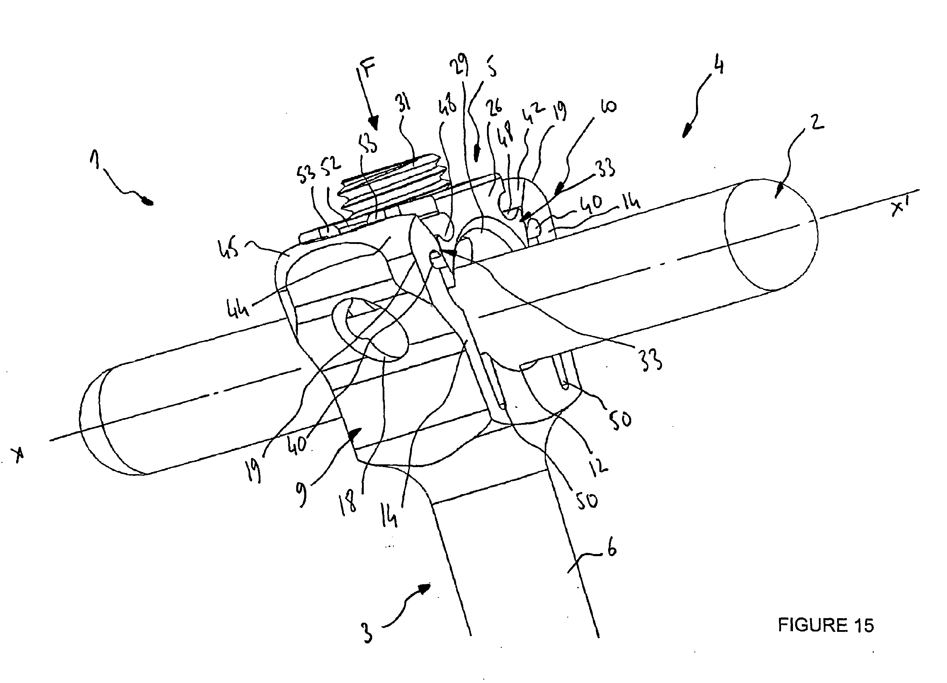

Chaque ergot 33, 34 comporte respectivement une partie d'accrochage 48, 49 délimitée par un profil en portion de cylindre permettant une coopération avec les parties d'accrochage 40, 41 de chaque dent 19, 20 lors de la mise en place de l'élément de blocage 5 dans la tête 8 de l'élément d'ancrage 3 (figure 15)

Chaque ergot 33, 34 présente respectivement un profil externe incliné 37, 38 permettant le glissement desdits ergots et l'écartement élastique des parois 9, 10 et donc des lames d'accrochage 14, 15 suivant la direction YY' afin de pouvoir réaliser l'assemblage de l'élément de blocage 5 avec l'élément d'ancrage osseux 3.

On note que chaque face opposée 27, 28 comporte en son milieu et entre les ergots 33, 34 un logement vertical 52 bordé latéralement par des nervures 53 permettant le guidage de l'élément de blocage 5 lors de son introduction dans la tête 8 de l'élément d'ancrage 3.

On constate que les parties d'accrochage 48, 49 sont fermées à l'opposé des faces latérales 25, 26 par l'intermédiaire de la nervure verticale 53 correspondante et disposée entre chaque ergot 33, 34.

Chaque nervure verticale 53 peut présenter un profil externe de forme quelconque, pourvu que son profil soit complémentaire à celui de la rainure 54 ménagé dans l'épaisseur de la face interne de chaque paroi verticale 9, 10 de la tête 8 de l'élément d'ancrage 3.

Les logements verticaux 51 et 52 présentent un profil en portion de cylindre complémentaire afin de permettre l'introduction d'un instrument afin d'assurer le retrait de l'élément de blocage 5 de la tête 8 de l'élément d'ancrage 3.

On a montré en figures 15 à 18 les différentes étapes permettant l'assemblage de l'élément de blocage 5 dans la tête 8 de l'élément d'ancrage 3 en vue de la fixation en rotation et en translation de la tige de liaison 2 dans chaque dispositif d'immobilisation 1 ancré dans le corps vertébral d'une vertèbre.

La tige de liaison 2 est positionnée à l'intérieur de l'ouverture centrale 11 de la tête 8 de l'élément d'ancrage osseux 3 avant l'introduction de l'élément de blocage 5.

La mise en place de l'élément de blocage 5 décrit ci-dessus dans la tête 8 de l'élément d'ancrage 3 est identique à celle décrite précédemment en figures 1 à 5.

L'élément de blocage 5 est positionné au-dessus de la tête 8 de l'élément d'ancrage osseux 3 de manière que les ergots 33, 34 d'une même face latérale 27, 28 viennent en appui contre les dents correspondantes 19, 20 d'une même paroi verticale 9, 10.

Une force de poussée F est effectuée suivant une direction sensiblement verticale à l'aide d'un instrument, non représenté, sur l'élément de blocage 5 afin que les ergots 33, 34 de chaque face latérale 27, 28 déforment latéralement les parois verticales 9, 10 et donc les lames d'accrochage élastiques 14, 15.

La déformation élastique des parois verticales 9, 10 s'effectue en direction de l'extérieur de la tête 8 du fait de la différence de dimensions prévues entre les ergots 33, 34 et les dents 19, 20.

L'introduction de l'élément de blocage 5 est facilitée par le fait que chaque ergot 33, 34 présente une partie inférieure à profil incliné 37, 38 qui glisse sur le profil externe de chaque dent 19, 20 des lames d'accrochage 14, 15 de chaque paroi élastique 9, 10.

La force de poussée F doit être suffisante pour que chaque partie d'accrochage 48, 49 des ergots 33, 34 vienne s'encliqueter avec la partie d'accrochage 40, 41 de chaque dent 19, 20 correspondante.

La retenue de l'élément de blocage 5 est obtenue par l'élasticité des parois verticales 9, 10 et donc des lames d'accrochage 14, 15 qui reviennent en position de repos après le passage des ergots 33, 34 sur les dents 19, 20 correspondantes.

La tige de liaison 2 est ajustée par coulissement dans une gouttière cylindrique constituée par le fond 12 présentant un profil en portion de cylindre de la tête 8 de l'élément d'ancrage osseux 3 et le logement 29 présentant un profil en portion de cylindre de l'élément de blocage 5.

La tige de liaison 2 est ensuite immobilisée en rotation et en translation par l'intermédiaire de la vis de serrage 31 qui est vissée à l'intérieur de l'alésage 30 de l'élément de blocage 5. La vis de serrage 31, sous l'effort de vissage, vient bloquer la tige de liaison 2 contre le fond 12 en portion de cylindre de l'ouverture centrale 11 de la tête 8 de l'élément d'ancrage 3.

Egalement, l'effort de serrage de la vis de pression 31 contre la tige de liaison 2 permet, par l'intermédiaire d'un déplacement vertical dirigé suivant une direction opposée à celle de ladite tige, de bloquer l'élément de blocage 5 dans la tête 8 de l'élément d'ancrage 3.

Le retrait de l'élément de blocage 5 est obtenu par l'intermédiaire d'un instrument qui est introduit dans les logements verticaux 51 et 52 afin d'écarter les parois verticales 9, 10 en direction de l'extérieur de la tête 8 de l'élément d'ancrage 3.

Il doit d'ailleurs être entendu que la description qui précède n'a été donnée qu'à titre d'exemple et qu'elle ne limite nullement le domaine de l'invention dont on ne sortirait pas en remplaçant les détails d'exécutions décrits par tout autre équivalent.

## Revendications

1. Dispositif d'immobilisation d'une tige de liaison (2) dans un élément d'ancrage osseux (3) d'un implant rachidien (4), **caractérisé en ce qu'**il comporte d'une part un élément d'ancrage osseux (3) pourvu d'une tête (8) comportant deux parois verticales (9, 10) délimitant une ouverture centrale (11) en forme de U, lesdites parois verticales (9, 10) comprenant des lames d'accrochage (14, 15) susceptibles de se déformer élastiquement sous un effort de poussée F, lesdites lames d'accrochage (14, 15) comportant respectivement dans leur partie supérieure une dent d'encliquetage (19, 20), et d'autre part un élément de blocage (5), venant entre lesdites lames d'accrochage élastiques (14, 15), comprenant des moyens de serrage (31) et des ergots (33, .34) coopérant respectivement avec une dent (19, 20) solidaire des lames élastiques (14, 15).

2. Dispositif d'immobilisation suivant la revendication 1 **caractérisé en ce que** l'ouverture centrale (11) comprenne un fond présentant un profil en portion de cylindre, chaque paroi verticale élastique (9, 10)'étant séparée du fond (12) de l'ouverture centrale (11) par une fente verticale (50) donnant une certaine élasticité à chaque paroi selon une direction YY', lesdites parois verticales élastiques (9, 10) comprenant respectivement à chaque extrémité un profil en forme de lame d'accrochage (14, 15) disposées l'une en face de l'autre et de part et d'autre de l'ouverture centrale (11), et que l'élément de blocage (5) comporte un logement (29) à profil en portion de cylindre, un alésage fileté (30) débouchant à l'intérieur du logement (29) et lesdits moyens de serrage comportant une vis de serrage (31) coopérant avec l'alésage fileté (30).

3. Dispositif d'immobilisation suivant la revendication 2 **caractérisé en ce que** chaque paroi verticale élastique (9, 10) comporte sur sa face interne et entre les lames d'accrochage (14, 15) un logement vertical (51) en portion de cylindre possédant de chaque côté des rainures (54).

4. Dispositif d'immobilisation suivant la revendication 2 **caractérisé en ce que** les lames d'accrochage (14, 15) de la tête (8) comportent respectivement dans leur partie supérieure une dent (19, 20) dont le profil d'accrochage (40, 41) est tourné en direction de l'intérieur de l'ouverture centrale (11).

5. Dispositif d'immobilisation suivant la revendication 4 **caractérisé en ce que** chaque dent (19, 20) comporte au-dessus de sa partie d'accrochage (40, 41) et en direction de l'ouverture (11) un profil externe incliné (42, 43) se prolongeant en direction de l'extérieur par un profil bombé (44, 45).

6. Dispositif d'immobilisation suivant la revendication 2 **caractérisé en ce que** l'élément de blocage (5) comporte une face inférieure (24) comprenant suivant une direction parallèle à l'axe XX' un logement (29) présentant un profil en portion de cylindre afin de coopérer avec la tige de liaison (2), une face supérieure (23) comprenant en son milieu un alésage fileté (30) débouchant à l'intérieur du logement (29) et dans lequel coopère une vis de serrage (31), et des faces latérales (25, 26 27, 28) parallèles deux à deux et dont deux au moins (27, 28) sont solidaires respectivement de deux ergots (33, 34) en forme de dent.

7. Dispositif d'immobilisation suivant la revendication 6 **caractérisé en ce que** chaque face latérale (27, 28) disposée dans un plan parallèle à l'axe XX' du logement (29) comporte deux ergots (33, 34) en forme de dent comprenant des parties d'accrochages (48, 49) séparées par un logement vertical (52) bordé latéralement par des nervures (53).

8. Dispositif d'immobilisation suivant la revendication 6 **caractérisé en ce que** les parties d'accrochage (48, 49) sont fermées à l'opposé des faces latérales (25, 26) par l'intermédiaire de la nervure verticale (53) correspondante.

9. Dispositif d'immobilisation suivant la revendication 1, **caractérisé en ce que** l'ouverture centrale (11) comprenne un fond (12) présentant un profil en portion de cylindre, chaque paroi verticale (9, 10) étant constituée d'une face centrale (13) bordée latéralement et de chaque côté par des lames élastiques (14, 15) séparées respectivement de ladite face centrale par des fentes verticales (16, 17), et que l'élément de blocage (5) comporte un logement (29) à profil en portion de cylindre, un alésage fileté (30) débouchant à l'intérieur du logement (29) et lesdits moyens de serrage comportant une vis de serrage (31) coopérant avec l'alésage fileté (30).

10. Dispositif d'immobilisation suivant la revendication 9, **caractérisé en ce que** la face centrale (13) de chaque paroi verticale (9, 10) est percée d'un trou (18) débouchant à l'intérieur de l'ouverture centrale (11) en forme de U.

11. Dispositif d'immobilisation suivant la revendication 9, **caractérisé en ce que** les lames élastiques (14, 15) de la tête (8) comportent respectivement dans leur partie supérieure une dent (19, 20) dont le profil externe (21, 22) est bombé et incliné.

12. Dispositif d'immobilisation suivant la revendication 9, **caractérisé en ce que** l'élément de blocage (5) présente une face inférieure (24) comportant suivant une direction parallèle à l'axe XX' de la tige de liaison (2) un logement (29) présentant un profil en portion de cylindre.

13. Dispositif d'immobilisation suivant la revendication 9, **caractérisé en ce que** l'élément de blocage (5) présente une face supérieure (23), opposée à celle inférieure (24), comportant en son milieu un alésage fileté (30) débouchant à l'intérieur du logement (29) et dans lequel coopère une vis de serrage (31).

14. Dispositif d'immobilisation suivant la revendication 9, **caractérisé en ce que** l'élément de blocage (5) présente une première paire de faces latérales opposées (25, 26) comportant respectivement au dessus du logement (29) une empreinte (32) destinée à coopérer avec un instrument pour la manipulation et la mise en place dudit élément de blocage (5) sur l'élément d'ancrage osseux (3).

15. Dispositif d'immobilisation suivant la revendication 9, **caractérisé en ce que** l'élément de blocage (5) présente une seconde paire de faces latérales opposées (27, 28) qui sont solidaires chacune de deux ergots (33, 34) disposés dans la largeur dudit élément de blocage et positionnées dans le prolongement de chaque face latérale (25, 26).

16. Dispositif d'immobilisation suivant la revendication 9, **caractérisé en ce que** chaque ergot (33, 34) comporte respectivement dans sa partie supérieure un pan incliné ou chanfrein (35, 36) dont la base inférieure est positionnée dans le plan contenant chacune desdites faces latérales (25, 26).

17. Dispositif d'immobilisation suivant la revendication 9, **caractérisé en ce que** chaque ergot (33, 34) comporte respectivement dans sa partie inférieure et à l'opposé des pans inclinés (35, 36) un profil arrondi (37, 38).

18. Dispositif d'immobilisation suivant la revendication 9, **caractérisé en ce que** la distance **d** séparant deux ergots (33, 34) est inférieure à celle prévue entre deux dents (19, 20) d'une même paroi verticale (9, 10) de l'élément d'ancrage osseux (3).

19. Dispositif d'immobilisation suivant la revendication 9, **caractérisé en ce que** la force de poussée **F** appliquée sur l'élément de blocage (5) permet, par l'intermédiaire des ergots (33, 34) et des fentes verticales (16, 17), la déformation latérale des lames élastiques (14, 15) en direction de la face centrale (13) de chaque paroi (9, 10) de l'élément d'ancrage osseux (3).

20. Dispositif d'immobilisation suivant la revendication 1, **caractérisé en ce qu'**il comprend : un élément d'ancrage osseux (3) pourvu d'une tête (8) comportant deux parois verticales tronquées (9, 10) délimitant une ouverture centrale (11) en forme de U dont le fond (12) présente un profil en portion de cylindre, chaque paroi verticale (9, 10) étant constituée d'une face centrale (13) bordée latéralement et de chaque côté par des lames élastiques (14, 15) séparées respectivement de ladite face centrale par des fentes verticales (16, 17), et l'élément de blocage (5) comportant un logement (29) à profil en portion de cylindre, un alésage fileté (30) débouchant à l'intérieur du logement (29), et lesdits moyens de serrage comportant une vis de serrage (31) coopérant avec l'alésage fileté (30) et des ergots (33, 34) qui coopèrent respectivement avec une dent (19, 20) solidaire des lames élastiques (14, 15).

21. Dispositif d'immobilisation suivant la revendication 20, **caractérisé en ce que** la tête (8) comporte deux parois verticales (9, 10) à profil tronqué disposées l'une en face de l'autre et dans des plans parallèles afin de délimiter une première ouverture centrale (11) en forme de U portée par l'axe XX' de la tige de liaison (2) et dont le fond (12) présente un profil en portion de cylindre et une seconde ouverture (39) perpendiculaire à l'axe XX' et à la première ouverture (11).

22. Dispositif d'immobilisation suivant la revendication 21 **caractérisé en ce que** les deux ouvertures perpendiculaires (11, 39) permettent de délimiter à chaque angle de la tête (8) des lames élastiques (14, 15) susceptibles de se déformer élastiquement sous un effort de poussée **F**.

23. Dispositif d'immobilisation suivant la revendication 22 **caractérisé en ce que** les lames élastiques (14, 15) de la tête (8) comportent respectivement dans leur partie supérieure une dent (19, 20) dont le profil d'accrochage (40, 41) est tourné en direction de l'intérieur de la seconde ouverture (39) et au-dessus de la face centrale (13) de chaque paroi verticale (9, 10).

24. Dispositif d'immobilisation suivant la revendication 23 **caractérisé en ce que** chaque dent (19, 20) comporte au-dessus de sa partie d'accrochage (40, 41) et en direction de l'ouverture (39) un profil externe incliné (42, 43) se prolongeant en direction de l'extérieur par un profil bombé (44, 45).

25. Dispositif d'immobilisation suivant la revendication 20 **caractérisé en ce que** l'élément de blocage (5) comporte une face inférieure (24) comprenant suivant une direction parallèle à l'axe XX' un logement (29) présentant un profil en portion de cylindre afin de coopérer avec la tige de liaison (2), une face supérieure (23) comprenant en son milieu un alésage fileté (30) débouchant à l'intérieur du logement (29) et dans lequel coopère une vis de serrage (31), et des faces latérales (25, 26, 27, 28) parallèles deux à deux et dont deux au moins (27, 28) sont solidaires respectivement de deux ergots (33, 34) en forme de dent.

26. Dispositif d'immobilisation suivant la revendication 25 **caractérisé en ce que** chaque ergot (33, 34) comporte une partie d'accrochage (48, 49) positionnée en retrait et à une certaine distance **d1** des faces latérales et opposées (25, 26) de l'élément de blocage (5).

## Claims

1. Device for immobilizing a connection rod (2) in a bone-anchoring element (3) of a spinal implant (4), **characterized in that** it comprises, on the one hand, a bone-anchoring element (3) provided with a head (8) comprising two vertical walls (9, 10) that delimit a U-shaped central opening (11), said vertical walls (9, 10) comprising fastening blades (14, 15) that are able to deform elastically under a pressure force F, said fastening blades (14, 15) comprising respectively, in their upper part, a snap-in tooth (19, 20) and, on the other hand, a blocking element (5) coming between said elastic fastening blades (14, 15), with tightening means (31) and lugs (33, 34) cooperating respectively with a tooth (19, 20) integral with the elastic blades (14, 15).

2. Immobilization device according to Claim 1, **characterized in that** the central opening (11) has a bottom (12) with the profile of a cylinder portion, each elastic vertical wall (9, 10) being separated from the bottom (12) of the central opening (11) by a vertical slot (50) giving a certain elasticity to each wall in a direction YY', said elastic vertical walls (9, 10) comprising respectively, at each end, a profile in the form of a fastening blade (14, 15) arranged facing one another and on opposite sides of the central opening (11), and **in that** the blocking element (5) comprises a seat (29) with the profile of a cylinder portion, a threaded bore (30) opening out inside the seat (29), and said tightening means comprising a tightening screw (31) that cooperates with the threaded bore (30).

3. Immobilization device according to Claim 2, **characterized in that** each elastic vertical wall (9, 10) comprises, on its inner face and between the fastening blades (14, 15), a vertical seat (51) in the form of a portion of a cylinder, with grooves (54) on each side.

4. Immobilization device according to Claim 2, **characterized in that** the fastening blades (14, 15) of the head (8) comprise respectively, in their upper part, a tooth (19, 20) whose fastening profile (40, 41) is oriented in the direction of the interior of the central opening (11).

5. Immobilization device according to Claim 4, **characterized in that** each tooth (19, 20) comprises, above its fastening part (40, 41) and in the direction of the opening (11), an inclined outer profile (42, 43) which is continued in the outward direction by a convexly rounded profile (44, 45).

6. Immobilization device according to Claim 2, **characterized in that** the blocking element (5) has a lower face (24) comprising, in a direction parallel to the axis XX', a seat (29) with the profile of a cylinder portion in order to cooperate with the connection rod (2), an upper face (23) comprising at its centre a threaded bore (30) which opens out inside the seat (29) and within which a tightening screw (31) cooperates, and lateral faces (25, 26, 27, 28) which are parallel in pairs and of which at least two (27, 28) are respectively integral with two tooth-shaped lugs (33, 34).

7. Immobilization device according to Claim 6, **characterized in that** each lateral face (27, 28) arranged in a plane parallel to the axis XX' of the seat (29) comprises two tooth-shaped lugs (33, 34) with fastening parts (48, 49) that are separated by a vertical seat (52) bordered laterally by ribs (53).

8. Immobilization device according to Claim 6, **characterized in that** the fastening parts (48, 49) are closed opposite the lateral faces (25, 26) by way of the corresponding vertical rib (53).

9. Immobilization device according to Claim 1, **characterized in that** the central opening (11) has a bottom (12) with the profile of a cylinder portion, each vertical wall (9, 10) being formed by a central face (13) bordered laterally and on each side by elastic blades (14, 15) that are respectively separated from said central face by vertical slots (16, 17), and **in that** the blocking element (5) comprises a seat (29) with the profile of a cylinder portion, a threaded bore (30) opening out inside the seat (29), and said tightening means comprising a tightening screw (31) that cooperates with the threaded bore (30).

10. Immobilization device according to Claim 9, **characterized in that** the central face (13) of each vertical wall (9, 10) is breached by a hole (18) opening out inside the U-shaped central opening (11).

11. Immobilization device according to Claim 9,
**characterized in that** the elastic blades (14, 15) of the head (8) comprise respectively, in their upper part, a tooth (19, 20) whose external profile (21, 22) is convexly rounded and inclined.

12. Immobilization device according to Claim 9, **characterized in that** the blocking element (5) has a lower face (24) which, in a direction parallel to the axis XX' of the connection rod (2), has a seat (29) with the profile of a cylinder portion.

13. Immobilization device according to Claim 9, **characterized in that** the blocking element (5) has an upper face (23) arranged opposite the lower one (24) and comprising at its centre a threaded bore (30) which opens out inside the seat (29) and within which a tightening screw (31) cooperates.

14. Immobilization device according to Claim 9, **characterized in that** the blocking element (5) has a first pair of opposite lateral faces (25, 26) which comprise respectively, above the seat (29), an impression (32) intended to cooperate with an instrument for manipulation and placement of said blocking element (5) on the bone-anchoring element (3).

15. Immobilization device according to Claim 9, **characterized in that** the blocking element (5) has a second pair of opposite lateral faces (27, 28) which are each integral with two lugs (33, 34) arranged within the width of said blocking element and positioned in the continuation of each lateral face (25, 26).

16. Immobilization device according to Claim 9, **characterized in that** each lug (33, 34) comprises respectively, in its upper part, an inclined flat or bevel (35, 36) whose lower base is positioned in the plane containing each of said lateral faces (25, 26).

17. Immobilization device according to Claim 9, **characterized in that** each lug (33, 34) comprises respectively, in its lower part, and opposite the inclined flats (35, 36), a rounded profile (37, 38).

18. Immobilization device according to Claim 9, **characterized in that** the distance d separating two lugs (33, 34) is less than that provided between two teeth (19, 20) of a same vertical wall (9, 10) of the bone-anchoring element (3).

19. Immobilization device according to Claim 9, **characterized in that** the pressure force F applied to the blocking element (5) permits, by way of the lugs (33, 34) and the vertical slots (16, 17), the lateral deformation of the elastic blades (14, 15) in the direction of the central face (13) of each wall (9, 10) of the bone-anchoring element (3).

20. Immobilization device according to Claim 1, **characterized in that** it comprises: a bone-anchoring element (3) provided with a head (8) comprising two truncated vertical walls (9, 10) that delimit a U-shaped central opening (11), of which the bottom (12) has the profile of a cylinder portion, each vertical wall (9, 10) being formed by a central face (13) bordered laterally and on each side by elastic blades (14, 15) that are separated respectively from said central face by vertical slots (16, 17), and the blocking element (5) comprising a seat (29) with the profile of a cylinder portion, a threaded bore (30) opening out inside the seat (29), and said tightening means comprising a tightening screw (31) cooperating with the threaded bore (30) and the lugs (33, 34) which cooperate respectively with a tooth (19, 20) integral with the elastic blades (14, 15).

21. Immobilization device according to Claim 20, **characterized in that** the head (8) comprises two vertical walls (9, 10) of truncated profile arranged opposite one another and in parallel planes, in order to delimit a first U-shaped central opening (11) carried by the axis XX' of the connection rod (2), and of which the bottom (12) has the profile of a cylinder portion, and a second opening (39) perpendicular to the axis XX' and to the first opening (11).

22. Immobilization device according to Claim 21, **characterized in that** the two perpendicular openings (11, 39) make it possible to delimit, at each angle of the head (8), elastic blades (14, 15) that are able to deform elastically under a pressure force F.

23. Immobilization device according to Claim 22, **characterized in that** the elastic blades (14, 15) of the head (8) comprise respectively, in their upper part, a tooth (19, 20) whose fastening profile (40, 41) is oriented in the direction of the interior of the second opening (39) and above the central face (13) of each vertical wall (9, 10).

24. Immobilization device according to Claim 23, **characterized in that** each tooth (19, 20) comprises, above its fastening part (40, 41) and in the direction of the opening (39), an inclined external profile (42, 43) which is continued in the outward direction by a convexly rounded profile (44, 45).

25. Immobilization device according to Claim 20, **characterized in that** the blocking element (5) has a lower face (24) comprising, in a direction parallel to the axis XX', a seat (29) with the profile of a cylinder portion in order to cooperate with the connection rod (2), an upper face (23) comprising at its centre a threaded bore (30) which opens out inside the seat (29) and within which a tightening screw (31) cooperates, and lateral faces (25, 26, 27, 28) which are parallel in pairs and of which at least two (27, 28) are respectively integral with two tooth-shaped lugs (33, 34).

26. Immobilization device according to Claim 25,
**characterized in that** each lug (33, 34) comprises a fastening part (48, 49) set back from and at a certain distance d1 from the lateral and opposite faces (25, 26) of the blocking element (5).

## Patentansprüche

1. Vorrichtung zum Festhalten eines Verbindungsstabs (2) in einem Knochenankerelement (3) eines Wirbelsäulenimplantats (4), **dadurch gekennzeichnet, dass** sie einerseits ein Knochenankerelement (3) aufweist, das mit einem Kopf (8) versehen ist, der zwei senkrechte Wände (9, 10) aufweist, die eine zentrale Öffnung (11) in U-Form abgrenzen, wobei die senkrechten Wände (9, 10) Anhängklingen (14, 15) aufweisen, die sich elastisch unter einer Schubkraft F verformen können, wobei die Anhängklingen (14, 15) jeweils in ihrem oberen Teil einen Einrastzahn (19, 20) aufweisen und andererseits ein Blockierelement (5), das zwischen die elastischen Anhängklingen (14, 15) kommt, die Spannmittel (31) und Dorne (33, 34) aufweisen, die jeweils mit einem Zahn (19, 20), der fest mit den elastischen Klingen (14, 15) verbunden ist, zusammenarbeiten.

2. Vorrichtung zum Festhalten nach Anspruch 1, **dadurch gekennzeichnet, dass** die zentrale Öffnung (11) einen Boden (12) aufweist, der ein Zylinderabschnittprofil aufweist, wobei jede elastische senkrechte Wand (9, 10) von dem Boden (12) der zentralen Öffnung (11) durch einen senkrechten Schlitz (50) getrennt ist, der jeder Wand entlang einer Richtung YY' eine bestimmte Elastizität verleiht, wobei die senkrechten elastischen Wände (9, 10) jeweils an jedem Ende ein Profil in Form einer Anhängklinge (14, 15) aufweisen, die einander gegenüberliegend und zu beiden Seiten der zentralen Öffnung (11) angeordnet sind, und dass das Blockierelement (5) eine Aufnahme (29) mit Zylinderabschnittprofil aufweist, wobei eine Gewindebohrung (30) in das Innere der Aufnahme (29) mündet, und wobei die Spannmittel eine Spannschraube (31) aufweisen, die mit der Gewindebohrung (30) zusammenwirkt.

3. Vorrichtung zum Festhalten nach Anspruch 2, **dadurch gekennzeichnet, dass** jede senkrechte elastische Wand (9, 10) auf ihrer Innenseite und zwischen den Anhängklingen (14, 15) eine senkrechte Aufnahme (51) in Zylinderabschnitt aufweist, die an jeder Seite Rillen (54) besitzt.

4. Vorrichtung zum Festhalten nach Anspruch 2, **dadurch gekennzeichnet, dass** die Anhängklingen (14, 15) des Kopfs (8) jeweils in ihrem oberen Teil einen Zahn (19, 20) aufweisen, dessen Anhängprofil (40, 41) zum Inneren der zentralen Öffnung (11) gekehrt ist.

5. Vorrichtung zum Festhalten nach Anspruch 4, **dadurch gekennzeichnet, dass** jeder Zahn (19, 20) über seinem Anhängteil (40, 41) und in Richtung der Öffnung (11) ein geneigtes externes Profil (42, 43) aufweist, das sich nach außen durch ein gewölbtes Profil (44, 45) verlängert.

6. Vorrichtung zum Festhalten nach Anspruch 2, **dadurch gekennzeichnet, dass** das Blockierelement (5) eine Innenseite (24) aufweist, die entlang einer Richtung parallel zur Achse XX' eine Aufnahme (29) aufweist, die ein Zylinderabschnittprofil aufweist, um mit dem Verbindungsschaft (2) zusammenzuwirken, wobei eine obere Seite (23) in ihrer Mitte eine Gewindebohrung (30) aufweist, die in das Innere der Aufnahme (29) mündet, und in der eine Spannschraube (31) zusammenwirkt, und seitliche Seiten (25, 26, 27, 28), die gepaart parallel sind und von welchen mindestens zwei (27, 28) jeweils fest mit zwei Dornen (33, 34) in Zahnform verbunden sind.

7. Vorrichtung zum Festhalten nach Anspruch 6, **dadurch gekennzeichnet, dass** jede seitliche Seite (27, 28), die in einer Ebene parallel zur Achse XX' der Aufnahme (29) angeordnet ist, zwei Dorne (33, 34) in Zahnform aufweist, die Anhängteile (48, 49) aufweisen, die von einer senkrechten Aufnahme (52), die seitlich durch Rippen (53) eingefasst ist, getrennt sind.

8. Vorrichtung zum Festhalten nach Anspruch 6, **dadurch gekennzeichnet, dass** die Anhängteile (48, 49) auf der den seitlichen Seiten (25, 26) entgegengesetzten Seite mittels der entsprechenden senkrechten Rippe (53) geschlossen sind.

9. Vorrichtung zum Festhalten nach Anspruch 1, **dadurch gekennzeichnet, dass** die zentrale Öffnung (11) einen Boden (12) aufweist, der ein Zylinderabschnittprofil aufweist, wobei jede senkrechte Wand (9, 10) aus einer zentralen Seite (13) besteht, die seitlich und zu jeder Seite von elastischen Klingen (14, 15) eingefasst ist, die jeweils von der zentralen Seite durch senkrechte Schlitze (16, 17) getrennt sind, und dass das Blockierelement (5) eine Aufnahme (29) mit Zylinderabschnittprofil aufweist, wobei eine Gewindebohrung (30) in das Innere der Aufnahme (29) mündet, und wobei die Spannmittel eine Spannschraube (31) aufweisen, die mit der Gewindebohrung zusammenwirkt.

10. Vorrichtung zum Festhalten nach Anspruch 9, **dadurch gekennzeichnet, dass** die zentrale Seite (13) jeder senkrechten Wand (9, 10) mit einem Loch (18) durchbohrt ist, das in das Innere der zentralen Öffnung (11) in U-Form mündet.

11. Vorrichtung zum Festhalten nach Anspruch 9, **dadurch gekennzeichnet, dass** die elastischen Klingen (14, 15) des Kopfs (8) jeweils in ihrem oberen Teil einen Zahn (19, 20) aufweisen, dessen externes Profil (21, 22) gewölbt und geneigt ist.

12. Vorrichtung zum Festhalten nach Anspruch 9, **dadurch gekennzeichnet, dass** das Blockierelement (5) eine untere Seite (24) aufweist, die entlang einer Richtung parallel zur Achse XX' des Verbindungsschafts (2) eine Aufnahme (29) aufweist, die ein Zylinderabschnittprofil aufweist.

13. Vorrichtung zum Festhalten nach Anspruch 9, **dadurch gekennzeichnet, dass** das Blockierelement (5) eine obere Seite (23) der unteren Seite (24) entgegengesetzt aufweist, wobei die obere Seite (23) in ihrer Mitte eine Gewindebohrung (30) enthält, die in das Innere der Aufnahme (29) mündet, und in der eine Spannschraube (31) zusammenwirkt.

14. Vorrichtung zum Festhalten nach Anspruch 9, **dadurch gekennzeichnet, dass** das Blockierelement (5) ein erstes Paar seitlicher entgegengesetzter Seiten (25, 26) aufweist, die jeweils über der Aufnahme (29) eine Prägung (32) aufweisen, die dazu bestimmt ist, mit einem Instrument zum Handhaben und Anbringen des Blockierelements (5) auf dem Knochenankerelement (3) zusammenzuwirken.

15. Vorrichtung zum Festhalten nach Anspruch 9, **dadurch gekennzeichnet, dass** das Blockierelement (5) ein zweites Paar seitlicher entgegengesetzter Seiten (27, 28) aufweist, die jeweils mit zwei Dornen (33, 34), die in der Breite des Blockierelements angeordnet und in der Verlängerung jeder seitlichen Seite (25, 26) positioniert sind, fest verbunden sind.

16. Vorrichtung zum Festhalten nach Anspruch 9, **dadurch gekennzeichnet, dass** jeder Dorn (33, 34) jeweils in seinem oberen Teil eine schiefe Ebene oder Abfasung (35, 36) aufweist, deren untere Basis in der Ebene, die jede der seitlichen Seiten (25, 26) enthält, positioniert ist.

17. Vorrichtung zum Festhalten nach Anspruch 9, **dadurch gekennzeichnet, dass** jeder Dorn (33, 34) jeweils in seinem unteren Teil und den schiefen Ebenen (35, 36) entgegengesetzt ein gerundetes Profil (37, 38) aufweist.

18. Vorrichtung zum Festhalten nach Anspruch 9, **dadurch gekennzeichnet, dass** die Entfernung d, die zwei Dorne (33, 34) trennt, kleiner ist als die, die zwischen den zwei Zähnen (19, 20) einer gleichen senkrechten Wand (9, 10) des Knochenankerelements (3) vorgesehen ist.

19. Vorrichtung zum Festhalten nach Anspruch 9, **dadurch gekennzeichnet, dass** die Schubkraft F, die an das Blockierelement (5) angelegt wird, über Dorne (33, 34) und senkrechte Schlitze (16, 17) das seitliche Verformen der elastischen Klingen (14, 15) in Richtung der zentralen Seite (13) jeder Wand (9, 10) des Knochenankerelements (3) erlaubt.

20. Vorrichtung zum Festhalten nach Anspruch 1, **dadurch gekennzeichnet, dass** sie Folgendes aufweist: ein Knochenankerelement (3), das mit einem Kopf (8) versehen ist, der zwei stumpfe senkrechte Wände (9, 10) aufweist, die eine zentrale Öffnung (11) in U-Form abgrenzen, deren Boden (12) ein Zylinderabschnittprofil aufweist, wobei jede senkrechte Wand (9, 10) aus einer zentralen Seite (13) besteht, die seitlich und auf jeder Seite durch elastische Klingen (14, 15) eingefasst ist, die jeweils von der zentralen Seite durch senkrechte Schlitze (16, 17) getrennt sind, und wobei das Blockierelement (5), das eine Aufnahme (29) mit Zylinderabschnittprofil aufweist, eine Gewindebohrung (30) aufweist, die in das Innere der Aufnahme (29) mündet, und wobei die Spannmittel eine Spannschraube (31) aufweisen, die mit der Gewindebohrung (30) zusammenwirkt, und Dorne (33, 34), die jeweils mit einem Zahn (19, 20), der fest mit den elastischen Klingen (14, 15) verbunden ist, zusammenwirken.

21. Vorrichtung zum Festhalten nach Anspruch 20, **dadurch gekennzeichnet, dass** der Kopf (8) zwei senkrechte Wände (9, 10) mit stumpfem Profil aufweist, die einander gegenüberliegend und in parallelen Ebenen angeordnet sind, um eine erste zentrale Öffnung (11) in U-Form, die von der Achse XX' des Verbindungsschafts (2) verbunden sind, abzugrenzen, und deren Boden (12) ein Zylinderabschnittprofil aufweist, sowie eine zweite Öffnung (39) senkrecht zur Achse XX' und zu der ersten Öffnung (11).

22. Vorrichtung zum Festhalten nach Anspruch 21, **dadurch gekennzeichnet, dass** die zwei senkrechten Öffnungen (11, 39) erlauben, an jedem Winkel des Kopfs (8) elastische Klingen (14, 15) abzugrenzen, die sich unter einer Schubkraft F elastisch verformen können.

23. Vorrichtung zum Festhalten nach Anspruch 22, **dadurch gekennzeichnet, dass** die elastischen Klingen (14, 15) des Kopfs (8) jeweils in ihrem oberen Teil einen Zahn (19, 20) aufweisen, dessen Anhängprofil (40, 41) in das Innere der zweiten Öffnung (39) und über der zentralen Seite (13) zu jeder senkrechten Wand (9, 10) gewandt ist.

24. Vorrichtung zum Festhalten nach Anspruch 23, **dadurch gekennzeichnet, dass** jeder Zahn (19, 20) über seinem Anhängteil (40, 41) und in Richtung der Öffnung (39) ein geneigtes externes Profil (42, 43) aufweist, das sich nach außen durch ein gewölbtes Profil (44, 45) verlängert.

25. Vorrichtung zum Festhalten nach Anspruch 20, **dadurch gekennzeichnet, dass** das Blockierelement (5) eine untere Seite (24) aufweist, die entlang einer Richtung parallel zu der Achse XX' eine Aufnahme (29) enthält, die ein Zylinderabschnittprofil aufweist, um mit dem Verbindungsschaft (2) zusammenzuarbeiten, wobei eine obere Seite (23) in ihrer Mitte eine Gewindebohrung (30) aufweist, die in das Innere der Aufnahme (29) mündet, und in welcher eine Spannschraube (31) zusammenwirkt, und seitliche Seiten (25, 26, 27, 28), die gepaart parallel sind und von welchen mindestens zwei (27, 28) jeweils fest mit zwei Dornen (33, 34) in Zahnform verbunden sind.

26. Vorrichtung zum Festhalten nach Anspruch 25, **dadurch gekennzeichnet, dass** jeder Dorn (33, 34) einen Anhängteil (48, 49) aufweist, der vertieft und in einer bestimmten Entfernung d1 von den seitlichen und entgegengesetzten Seiten (25, 26) des Blockierelements (5) positioniert ist.
